# EUROPEAN PATENT APPLICATION

(11) **EP 1 717 350 A1**
(43) Date of publication of application: **02.11.2006**
(21) Application number: 05710526.4
(22) Date of filing: 16.02.2005
(51) Int. Cl.: C23F 11/00, B32B 15/08, C07C 243/38, C07C 251/76, C09D 5/08, C09D 201/00

(54) **ANTIRUST COMPOSITION**

(30) Priority: 16.02.2004 JP 2004037782
(71) Applicant: Otsuka Chemical Co., Ltd., Osaka-shi, Osaka 540-0021 (JP)
(72) Inventor: SONOGI, Ken, c/o Otsuka Chemical Co., Ltd., Tokushima-shi, Tokushima 771-0193 (JP); NABESHIMA, Akihiro, c/o Otsuka Chemical Co., Ltd., Tokushima-shi, Tokushima 771-0193 (JP)
(74) Representative: Ritthaler, Wolfgang
(86) International application number: PCT/JP2005/002823
(87) International publication number: WO 2005/078157

(57) **Abstract**

A rust preventive agent which contains, as an active component, at least one species selected from a compound of the formula (1) or (2) and a salt thereof; a rust preventing method and a rust preventive resin composition using the rust preventive agent.

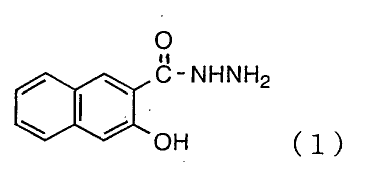

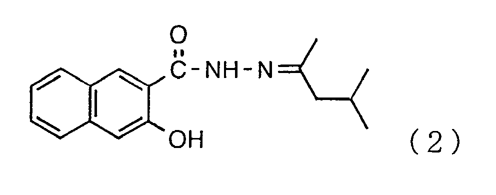

## Description

### Field of the Invention

The present invention relates to a rust preventive agent, a rust preventing method and a rust preventive composition.

### Background Art

A hydroxynaphthoic acid hydrazide compound is known. For example, hydroxynaphthoic acid hydrazide and a hydrazone compound of methyl isobutyl ketone prepared from the hydroxynaphthoic acid hydrazide compound are known as a heat generation preventive against rubbers for tires or the like (e.g., patent literature 1 or 2), and as an antioxidant for natural rubbers or synthetic rubbers (e.g. patent literature 3). However, it is unknown that these hydrazide compounds are effective in preventing the rust of metals.
[Patent literature 1] JP1995-57828B
[Patent literature 2] JP1999-292834A
[Patent literature 3] WO98/44040

It is an object of the present invention to provide a rust preventive agent which exhibits and maintains an effect of preventing the rust of metals even under severe environments, the rust preventive agent being especially effective in preventing the rust of copper, zinc and iron and alloys thereof.

### Disclosure of the Invention

The present invention includes the followings.
1. A rust preventive agent which contains, as an active component, at least one species selected from a compound of the formula (1) or (2) and a salt thereof. 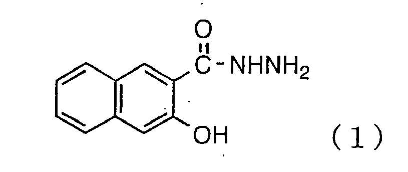 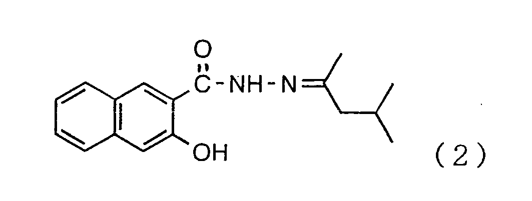
2. A rust preventive agent described in an item 1 wherein the salt of the compound of the formula (1) or (2) is an alkali metal salt.
3. A rust preventing method which is characterized in that the surface of the metal or alloy is coated with the rust preventive agent described in an item 1 or 2.
4. A rust preventive resin composition which contains 0.001 to 10 parts by weight of the rust preventive agent described in an item 1 or 2 per 100 parts by weight of a resin.
5. A rust preventive resin composition described in an item 4 wherein the resin is at least one species selected from the group consisting of epoxy-based resins, polyolefin-based resins, chlorinated polyolefin-based resins, vinyl chloride-based resins, alkyd-based resins, guanamine-based resins, phenol-based resins, fluoroplastic resins, polyacrylonitrile-based resins, polystyrene-based resins, polyacetal-based resins, polyamide-based resins, (meth)acrylic ester-based resins, polyimide-based resins, polyether ether ketone-based resins, polyethylene oxide-based resins, polyester-based resins, polyvinyl acetate-based resins, polyvinyl alcohol-based resins, polyvinyl ether-based resins, polyphenylene ether-based resins, polyphenylene oxide-based resins, polymethylpentene-based resins, polyurethane-based resins, melamine-based resins, urea-based resins, polycarbonate-based resins, furan-based resins, silicone-based resins, ionomer-based resins, polyisocyanate-based resins, polyterpene-based resins, and at least one species selected from copolymers thereof.
6. A rust preventive resin composition described in an item 4 wherein the resin is at least one species selected from the group consisting of epoxy-based resins, polyolefin-based resins, vinyl chloride-based resins, polyacrylonitrile-based resins, polystyrene-based resins, polyamide-based resins, (meth)acrylic ester-based resins, polyether ether ketone-based resins, polyester-based resins, polyvinyl acetate-based resins, polyurethane-based resins, polycarbonate-based resins, and at least one species selected from copolymers thereof.
7. A method of preventing the rust of a metal or alloy thereof, the method being characterized in that the surface of the metal or alloy thereof is coated with or applied with the rust preventive resin composition described in any one of items 4 to 6.
8. A conductive body formed by being coated with the rust preventive resin composition described in any one of items 4 to 6.
9. A rust preventive coating composition which contains the rust preventive resin composition described in any one of items 4 to 6.
10. A rust preventing method described in an item 3 or 7 in which the metal or alloy is one made of at least one species selected from copper, zinc and iron.

According to the invention, it was found that a specific type of hydroxynaphthoic acid hydrazide is markedly useful as a rust preventive agent for a metal and alloy thereof.

The rust preventive agent of the present invention contains, as an active component, at least one species selected from a compound of the formula (1) or (2) and a salt thereof.

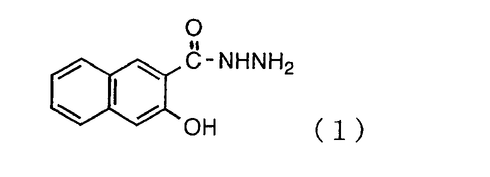

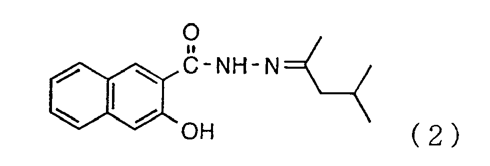

The compound of the formula (1) or (2) can be used singly or in combination. The compound of the formula (1) or (2) even in the form of a salt can achieve a rust preventive effect.

Examples of the salt are salts of alkali metals such as sodium, potassium, lithium or the like and salts of alkaline earth metals such as calcium, magnesium or the like. The salts can be easily prepared by mixing the compound of the formula (1) or (2) with a base such as alkali metal hydroxide, e.g. sodium hydroxide, potassium hydroxide, lithium hydroxide or the like or alkaline earth metal hydroxide, e.g. calcium hydroxide, magnesium hydroxide or the like. These bases can be used singly or in combination. When the compound of the formula (1) or (2) is used in the form of a salt, the compound can be used in a state of an aqueous solution. Hence it is preferred.

The rust preventive agent of this invention can exhibit a rust preventing effect by coating the surface of a metal or alloy thereof with the agent. Metals which can be prevented from the rust by the rust preventive agent of the invention are not limited insofar as the metals are those which need to be prevented from the rust. Such metals include, e.g., copper, zinc, iron, etc. and may be alloys containing at least one of these metals.

The rust preventive agent of the invention can be used by the following method. The rust preventive agent is dissolved in a suitable solvent to give a solution. The solution is sprayed or applied with a spray or a roll coater to the surface of ingots, chips or molded products formed from copper, zinc, iron or the like or they are immersed in the solution.

Useful solvents for dissolving the rust preventive agent are, for example, methanol, ethanol, isopropanol or like alcohols, dichloromethane, dichloroethane, chloroform, carbon tetrachloride or like halogenated hydrocarbons, methyl acetate, ethyl acetate or like esters, diethyl ether, tetrahydrofuran or like ethers, N,N-dimethylformamide or like amides and organic solvents and water which can be usually used. These solvents can be used in combination.

The concentrations of the compound of the formula (1) and/or (2) and the salt to be used can be suitably determined. Usually the concentration in total is 10 ppm or more, preferably 10 to 10000 ppm, more preferably 100 to 1000 ppm.

The temperature for applying the rust preventive agent can be properly determined, and is usually 0 to 100°C, preferably room temperature to about 80°C.

The amount of the rust preventive agent of the invention is not limited and is sufficient insofar as the agent can uniformly cover the entire surface of the substrate. More specifically 0.01 to 500 g, preferably about 0.1 to about 50 g for 1 m² of area to be applied with the agent.

When the rust preventive agent of the invention is applied to the surface of a product molded from copper, zinc, iron or like metal or alloys thereof, the molded product to be coated with a coating composition can be transported or stored for a prolonged period. That is, the use of the agent contributes to rational improvements of manufacturing process.

The rust preventive agent of the invention may be incorporated into a resin useful for covering metals or alloys to give a rust preventive resin composition.

When the compound of the formula (1) and/or (2) according to the invention is incorporated, the deterioration of resin can be prevented as clear from rust prevention effect test 1-2 and 2-1 in Examples 4 and 5 to be set forth later.

Resins to be used in the present invention are not limited and include thermoplastic resins, thermosetting resins, etc. For example, the following resins can be used. However, natural rubber and synthetic rubber to be used for tires, etc. are not included.

Examples of useful resins are epoxy-based resins, polyethylene, polypropylene, polybutylene, and copolymers thereof and like polyolefin-based resins, chlorinated polyethylene, chlorinated polypropylene and like chlorinated polyolefin-based resins, polyvinyl chloride, polyvinylidene chloride and like vinyl chloride-based resins, alkyd-based resins, guanamine-based resins, phenol-based resins, tetrafluoroethylene (PTFE), fluoroethylene-polypropylene copolymer (FEP), and fluoroplastic resins, polyacrylonitrile-based resins, polystyrene-based resins, polyacetal-based resins, nylon 6, 11, 12, 46, 66, 610 or 612 and polyamide-based resins such as copolymers thereof, polymethyl acrylate, polymethyl methacrylate, ethylene-ethylacrylate copolymers and like (meth)acrylic ester-based resins, thermoplastic polyimide, polyether imide and like polyimide-based resins, polyether ether ketone-based resins, polyethylene oxide-based resins, polyethylene terephthalate (PET), polybutylene terephthalate (PBT), and copolymers thereof and like polyester-based resins, polyvinyl acetate-based resins, polyvinyl alcohol-based resins, polyvinyl ether-based resins, polyphenylene ether-based resins, polyphenylene oxide-based resins, polymethylpentene-based resins, polyurethane-based resins, melamine-based resins, urea-based resins, polycarbonate-based resins, furan-based resins, silicone-based resins, ionomer-based resins, polyisocyanate-based resins, polyterpene-based resins, and copolymers thereof. Among them, it is preferred to use epoxy-based resins, polyolefin-based resins, vinyl chloride-based resins, polyacrylonitrile-based resins, polystyrene-based resins, polyamide-based resins, (meth)acrylic ester-based resins, polyether ether ketone-based resins, polyester-based resins, polyvinyl acetate-based resins, polyurethane-based resins, polycarbonate-based resins and copolymers thereof. The resin may be modified for adding a specific property. These resins can be used singly or may be used as blended in combination.

The ratio of the rust preventive agent of the invention and these resins to be used may be 0.001 to 10 parts by weight, preferably 0.01 to 1 part by weight, of the rust preventive agent, per 100 parts by weight of the resin. If the amount of the agent is less than 0.001 part by weight, the effect is less than expected, whereas more than 10 parts scarcely exhibits the increased effect.

The rust preventive resin composition of the invention may contain additives such as plasticizers, coloring agents, antioxidants, crosslinking assistants, UV absorbers, processing assistants, stabilizers and others when required insofar as they do not deteriorate the intended effect.

The rust preventive resin composition of the invention can be easily made by mixing the components uniformly using a Banbury mixer, a pressure kneader, twin-screw kneader, such as those usually used.

The rust preventive resin composition of the invention thus prepared is applied to the surrounding of a conductor using a single-screw extruder or the like, whereby a conductor can be produced, e.g. electric wires or cables for automobiles, those for telephones, those for motive powers and those for heaters in heating carpets. Examples of the conductors are copper wires made of copper and/or alloys and steel wires made of iron and/or alloys thereof.

Molded products such as a plug and other products can be produced by use of an injection molding machine. Products can be molded according to molding methods such as extruding, injection, blowing, calendering, etc.

The rust preventive resin composition of the invention is applicable to metals and alloys thereof which require rust preventing effects, and can be used in the form of building materials, rust preventing coating compositions for building or construction, automotive rust preventing coating compositions, coating compositions for plating, or heavily rust-preventing coating compositions.

### BEST MODE FOR CARRYING OUT THE INVENTION

The invention will be described in more detail with reference to Examples to which, however, the invention is not limited. The parts are all by weight.

### Example 1 (Rust Prevention Against Copper)

A copper sheet (25 X 50 X 1 mm) was polished with a piece of abrasive cloth, degreased with acetone, and dried.

The test compounds are 3-hydroxy-2-naphthoic acid hydrazide (compound 1), 3-hydroxy-N'-(1,3-dimethylbutylidene)-2-naphthoic acid hydrazide (compound 2), and 3-hydroxy-N'-isopropylidene-2-napthoic acid hydrazide (comparative compound 1).

Each compound was dissolved in an aqueous solution of molar equivalent sodium hydroxide to give 100 ppm, 500 ppm and 1000 ppm on addition of deionized water, whereby each test solution was obtained. Comparative solutions were an aqueous solution of benzotriazole as a rust preventive for copper, and deionized water as a blank.

Each copper sheet was immersed in each test solution for 10 minutes, washed with deionized water and dried at 50°C, followed by standing in a thermostatic chamber at 90°C of 90% in humidity.

After 15 days, the electrical resistance was measured by a four-terminal four-probe method [constant current-applied method] (measuring probe: ASP probe, measuring equipment: Loresta MP-T350), product of Mitsubishi Chemical Corp.

The electrical resistance was measured at optional positions on both sides at 10 places on one side of the copper sheet. Average values of 10 points other than 5 high points and 5 low points were determined. Relative values were found when the electrical resistance of the blank was taken as 1. The lower the values, the higher the rust prevention level.

**[Table 1]**

| | Concentration 100 ppm | | Concentration 500 ppm | | Concentration 1000 ppm | |
|---|---|---|---|---|---|---|
| | *1 | *2 | *1 | *2 | *1 | *2 |
| Compound 1 | 0.43 | ⊚ | 0.34 | ⊚ | 0.29 | ⊚ |
| Compound 2 | 0.45 | ⊚ | 0.33 | ⊚ | 0.32 | ⊚ |
| Comparative Compound 1 | 0.73 | Δ | 0.88 | × | 1.04 | × |
| benzotriazole | 0.9 | × | 0.73 | Δ | 0.47 | ⊚ |
| blank | 1 | × | 1 | × | 1 | × |

| | | | | | | |
|---|---|---|---|---|---|---|
| *1 : Relative electrical resistance *2 : Evaluation | | | | | | |

Evaluation Standards
A: Less than 0.5
B: More than 0.5/less than 0.7
C: More than 0.7/less than 0.8
D: More than 0.8

### Example 2 (Rust prevention against zinc)

A zinc sheet (25 X 50 X 1 mm) was polished with a piece of water-resistant abrasive paper, degreased with acetone, and dried.

The test compounds are 3-hydroxy-2-naphthoic acid hydrazide (compound 1), 3-hydroxy-N'-(1,3-dimethylbutylidene)-2-naphthoic acid hydrazide (compound 2), salicylic acid hydrazide (comparative compound 1), 3-hydroxy-N'-isopropylidene-2-napthoic acid hydrazide (comparative compound 2), N'-benzylidene-3-hydroxy-2-napthoic acid hydrazide (comparative compound 3), 3-hydroxy-N'-(1-phenylethylidene)-2-naphthoic acid hydrazide (comparative compound 4).

Each compound was dissolved in an aqueous solution of molar equivalent sodium hydroxide to provide 100 ppm, 500 ppm or 1000 ppm of a solution on addition of deionized water, whereby each test solution was obtained.

Comparative test solutions were an aqueous solution of 1,2,4-triazole as a rust preventive for zinc, and deionized water as a blank.

Each zinc sheet was immersed in each test solution for 10 minutes, washed with deionized water, and dried at 50°C, followed by standing in a thermostatic chamber of 60°C, 90% in humidity.

After 12 days, the electrical resistance was measured and the results were obtained and evaluated in the same manner as in Example 1. The results are shown in Table 2.

**[Table 2]**

| | Concentration 100 ppm | | Concentration 500 ppm | | Concentration 1000 ppm | |
|---|---|---|---|---|---|---|
| | *1 | *2 | *1 | *2 | *1 | *2 |
| Compound 1 | 0.63 | ○ | 0.61 | ○ | 0.64 | ○ |
| Compound 2 | 0.46 | ⊚ | 0.24 | ⊚ | 0.28 | ⊚ |
| Comparative Compound 1 | 1.20 | × | 1.33 | × | 1.03 | × |
| Comparative Compound 2 | 0.89 | × | 2.11 | × | 8.11 | × |
| Comparative Compound 3 | 1.14 | × | 0.8 | × | 1.0 | × |
| Comparative Compound 4 | 0.86 | × | 0.8 | × | 0.97 | × |
| 1,2,4-triazole | 0.56 | ○ | 0.51 | ○ | 0.54 | ○ |
| blank | 1 | × | 1 | × | 1 | × |

| | | | | | | |
|---|---|---|---|---|---|---|
| *1 : Relative electrical resistance *2 : Evaluation | | | | | | |

### Example 3 (Rust prevention against iron)

An iron sheet (25 X 50 X 1 mm) was polished with a piece of abrasive cloth.

The test compound was 3-hydroxy-N'-(1,3-dimethylbutylidene)-2-naphthoic acid hydrazide (compound 2). The compound was dissolved in an aqueous solution of molar equivalent sodium hydroxide to give an aqueous solution of sodium salt and deionized water was added to provide 500 ppm of test solution.

500 ppm of an aqueous solution of 1-hydroxybenzotriazole as a rust preventive against iron and deionized water (blank) as a comparative test solution were provided.

Each iron sheet was immersed in each test solution for 10 minutes, washed with deionized water and dried at 50°C, followed by standing in a thermostatic chamber of 60°C, 90% in humidity.

After 2 days, the electrical resistance was measured. The results were obtained and evaluated in the same manner as in Example 1. The results are shown in Table 3.

**[Table 3]**

| | Relative electrical resistance | Evaluation |
|---|---|---|
| Compound 2 | 0.0545 | ⊚ |
| 1-hydroxybenzotriazole | 0.1091 | ⊚ |
| blank | 1 | × |

### Example 4 (Rust prevention effect using a resin composition)

### (1) Production of resin sheet

Added to 100 parts of UBE Nylon 3014B (product of Ube Industries Ltd., polyamide-based resins) were 0.25 part of the test compound and 1.0 part of a heat resistance agent (Similizer GA80, product of Similizer GA80, product of Sumitomo Chemical Co, Ltd.). The mixture was kneaded with a twin-screw kneader (220°C, 5 minutes). The particles were crushed to give pellets. The pellets were made into a 1-mm thick rust preventive containing-resin sheet by a vacuum press machine. The test compounds were 3-hydroxy-N'-(1,3-dimethylbutylidene)-2-naphthoic acid hydrazide (compound 2), and benzotriazole or 1,10-bis(N-salicyloylamino)dodecanediamide.

A resin sheet (blank) was produced without using a test compound.

### (2) Rust prevention effect test 1-1

A copper sheet (100 X 5 X 0.5 mm) was sandwiched between two resin sheets (100 X 10 X 1.0 mm) and fixed with clips such that the resin sheets and the copper sheet were kept in close contact with each other. Then the copper sheet was allowed to stand in a thermostatic chamber at 140°C. Twenty days later, the electrical resistance of the copper sheet disengaged from the resin sheet was measured.

The electrical resistance was measured at optional positions on both sides at 10 places on one side of the copper sheet. An average value of 10 points other than 5 high points and 5 low points were determined. The results are shown in Table 4.

**[Table 4]**

| | Relative electrical resistance |
|---|---|
| Compound 2 | 0.13 |
| benzotriazole | 0.26 |
| 1,10-bis(N-salicyloylamino)dodecanediamide | 0.3 |
| blank | 1 |

### (3) Rust prevention effect test 1-2

After the rust prevention effect test 1-1, each resin sheet removed from the copper sheet was subjected to a tensile tester (reversed tensile and compression load frame method/Tensilon universal tester RTC-1310A [A and D product]) to measure a maximum spot stress (MPa).

The measurement results were evaluated in terms of a reducing ratio corresponding to maximum spot stress of each resin sheet produced in (1). The results are shown in Table 5.

**[Table 5]**

| | reducing ratio (%) |
|---|---|
| Compound 2 | 3.5 |
| benzotriazole | 23.0 |
| 1,10-bis(N-salicyloylamino)dodecanediamide | 20.6 |
| blank | 43.1 |

### Example 5 (Rust prevention effect test with resin composition 2)

### (1) Production of resin sheet.

Added to 100 parts of polyvinyl chloride S1001 (product of Kaneka Corp., polymerization degree 1050, vinyl chloride-based resin) were 50 parts of diisononyl phthalate, 30 parts of calcium carbonate, 5 parts of clay #33 (product of Tsuchiya Kaolin Co., Ltd.), 4 parts of tribasic lead sulphate, 0.5 part of dibasic lead stearate, 0.5 part of lead n-stearate, 0.05 part of dibenzoylmethane, and 0.05 part of the test compound. The mixture was kneaded with a roll kneader (160°C, 5 minutes). Thereafter the sheet was cut into pieces (3 cm square). The pieces were molded with a vacuum press machine to give 1-mm thick rust preventive agent-containing resin sheet.

The test compounds are 3-hydroxy-N'-(1,3-dimethylbutylidene)-2-naphthoic acid hydrazide (compound 2), benzotriazole and 1,10-bis(N-salicyloylamino)dodecanediamide.

A resin sheet free of the test compound (blank) was produced.

### (2) Rust prevention effect test 2-1

Each resin sheet thus prepared (100 X 10 X 1.0 mm) was sandwiched between two copper foils and fixed together with a vacuum press machine. Then the copper foils were allowed to stand in a thermostatic chamber at 60°C and 90% in humidity. Two months later, the copper foils at one end of each resin sheet was partly separated. A spring scale was attached to the copper foil end and was pulled so that the copper foils were slowly removed from the resin sheet. Then the maximum value shown by the spring scale was taken as the peel strength. The results are indicated in terms of relative value when the value of blank was 1. The lower the value, the less affected the copper foils are. The results are shown in Table 6.

**[Table 6]**

| | Relative peel strength |
|---|---|
| Compound 2 | 0.71 |
| benzotriazole | 0.91 |
| 1,10-bis(N-salicyloylamino)dodecanediamide | 0.93 |
| blank | 1 |

### (3) Rust prevention effect test 2-2

In rust prevention effect test 2-2, the electrical resistance of the copper foils separated from the resin was measured.

The electrical resistance was measured at optional positions on both sides of 10 places on one side of the copper foil. Average values of 10 points other than 5 high points and 5 low points were determined. Relative values were found when the electrical resistance of the blank was taken as 1. The results are shown in Table 7.

**[Table 7]**

| | Relative electrical resistance |
|---|---|
| Compound 2 | 0.47 |
| benzotriazole | 0.63 |
| 1,10-bis(N-salicyloylamino)dodecanediamide | 0.74 |
| blank | 1 |

### Example 6 (Rust prevention effect with resin composition 3)

### (1) production of resin sheet

Added to 100 parts of Sumicasen L-430 (Sumitomo Chemical Co., Ltd., polyethylene-based resins) was 1.0 part of the test compound, a heat resistance agent (Similizer GA80, product of Sumitomo Chemical Co., Ltd.). The mixture was kneaded with a roll kneader (155°C, 5 minutes) to give a resin sheet. The obtained sheet was crushed, and the crushed product was made into pellets. The pellets were made into a 1-mm thick rust preventive containing-resin sheet by a vacuum press machine.

The test compounds were 3-hydroxy-N'-(1,3-dimethylbutylidene)-2-naphthoic acid hydrazide (compound 2), and N,N'-bis[3-(3,5-di-tert-butyl-4-hydroxyphenyl)propionyl]hydrazine, which were added in the amounts shown in Table 8.

Resin sheets free of test compounds (blanks) were prepared.

### (2) Rust prevention effect test 3-1

Each resin sheet (100 X 10 X 0.1 mm) was sandwiched between two copper foils and attached together with a press machine. Then the foils were allowed to stand in a thermostatic chamber at 120°C. Three months later, the electrical resistance of copper foil removed from each resin was measured.

The electrical resistance was measured at 10 positions of copper foil attached to the resin, i.e. 20 positions in total. An average value of 10 points other than 5 higher points and 5 lower points was determined. The relative value was evaluated when the electrical resistance of the blank was 1. The results are shown in Table 8.

**[Table 8]**

| | Amount (part) | Relative electrical resistance |
|---|---|---|
| Compound 2 | 0.25 | 0.45 |
| | 0.125 | 0.49 |
| | 0.025 | 0.53 |
| N,N'-bis[3-(3,5-di-tert-butyl-4-hydroxyphenyl)propionyl]hydrazine | 0.25 | 1.03 |
| blank | 0 | 1 |

### Example 7 (Preparation of rust preventive coating composition)

A rust preventive coating composition was prepared by mixing in a 4-neck flask 100 parts of Everl EP-F101 (product of KURARAY Co., Ltd., ethylene-vinl alcohol copolymer resin with an ethylene content 32 mole%) and 400 parts by weight of a mixed solvent comprising isopropyl alcohol : water= 2 : 1 (wt ratio). The mixture was stirred at 75°C for 3 hours to give a stable resin solution (solids 20%). Added to 500 parts of the resin solution were 200 parts of Snowtex (produced by Nissan Chemical Industries, Ltd., an aqueuos dispersion of colloidal silica, solids 20%, average particle size of silica: about 20 nm) and 3 parts of 3-hydroxy-2-naphthoic acid hydrazide (compound 1) and the mixture was stirred to give a rust preventive coating composition (solids 20%).

A rust preventive coating composition was prepared in the same manner as above except that 3-hydroxy-N'-(1,3-dimethylbutylidene)-2-naphthoic acid hydrazide (compound 2) was used in place of the compound 1.

### INDUSTRIAL APPLICABILITY

The present invention provides a rust preventive agent which exhibits and maintains an effect of preventing the rust of metals even under severe environments, the rust preventive agent being especially effective in preventing the rust of copper, zinc and iron and alloys thereof. Further, the present invention provides a rust preventing method and a rust preventive resin composition using the rust preventive agent.

## Claims

1. A rust preventive agent which contains, as an active component, at least one species selected from a compound of the formula (1) or (2) and a salt thereof.

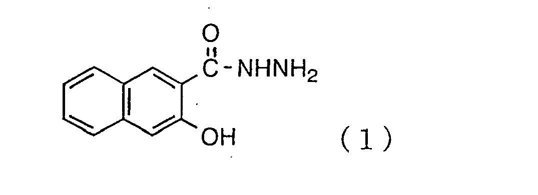

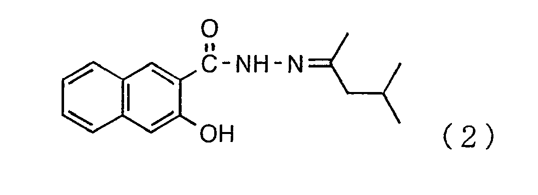

2. A rust preventive agent according to claim 1 wherein the salt of the compound of the formula (1) or (2) is an alkali metal salt.

3. A rust preventing method which is **characterized in that** the surface of the metal or alloy is coated with the rust preventive agent of claim 1 or 2.

4. A rust preventive resin composition which contains 0.001 to 10 parts by weight of the rust preventive agent of claim 1 or 2 per 100 parts by weight of a resin.

5. A rust preventive resin composition according to claim 4 wherein the resin is at least one species selected from the group consisting of epoxy-based resins, polyolefin-based resins, vinyl chloride-based resins, polyacrylonitrile-based resins, polystyrene-based resins, polyamide-based resins, (meth)acrylic ester-based resins, polyether ether ketone-based resins, polyester-based resins, polyvinyl acetate-based resins, polyurethane-based resins, polycarbonate-based resins, and at least one species selected from copolymers thereof.

6. A method of preventing the rust of a metal or alloy thereof, the method being **characterized in that** the surface of the metal or alloy thereof is coated with or applied with the rust preventive resin composition of claim 4 or 5.

7. A conductive body formed by being coated with the rust preventive resin composition of claim 4 or 5.

8. A rust preventive coating composition which contains the rust preventive resin composition of claim 4 or 5.

9. A rust preventing method according to claim 3 or 6 in which the metal or alloy is one made of at least one species selected from copper, zinc and iron.
